# EUROPEAN PATENT APPLICATION

(11) **EP 3 278 688 A1**
(43) Date of publication of application: **07.02.2018**
(21) Application number: 15887884.3
(22) Date of filing: 19.11.2015
(51) Int. Cl.: A45D 44/00, A45D 44/22, A61K 8/02, B41M 5/025, B41M 5/00, B41M 1/10

(54) **BASE SHEET FOR MASK PACK AND MASK PACK USING SAME**

(30) Priority: 02.04.2015 KR 20150046705; 13.07.2015 KR 20150099047
(71) Applicant: Genic Co., Ltd., Seoul 06752 (KR)
(72) Inventor: YOO, Hyun-Oh, Seoul 06544 (KR); LEE, Sang-Hun, Seoul 07630 (KR)
(74) Representative: Vidon Brevets & Stratégie
(86) International application number: PCT/KR2015/012472
(87) International publication number: WO 2016/159477

(57) **Abstract**

The present invention relates to a base sheet for a mask pack and a mask pack using the same and, more specifically, to: a base sheet for a mask pack, comprising a base sheet and a single layer image printing part formed on at least one surface of the base sheet; and a mask pack comprising the base sheet for a mask pack and a cosmetic composition layer. The base sheet for a mask pack of the present invention can improve a user's ease of use by including various types of images, can obtain an enhanced adhesion to a cosmetic composition layer, and can further implement a mask pack composition including various components according to each image section, and display the same to a user.

## Description

### [Technical Field]

The present disclosure relates to a base sheet for a mask pack and a mask pack using the mask pack base sheet, and the mask pack base sheet includes an image having various shapes.

### [Background Art]

Along with the increasing interest in beauty treatment in modern society, mask packs have been widely used for supplying nutrients and moisture to the face and maintaining the skin elasticity.

In general, mask packs, made by impregnating an absorptive base material such as a nonwoven fabric with cosmetic ingredients such as a beauty wash or an essence, are attached to the skin to transfer the cosmetic ingredients to the skin, and are then detached from the skin after a certain period of time.

In addition, other mask packs such as hydrogel mask packs, made by forming a cosmetic composition layer on one or both sides of a base sheet (support structure), are used to transfer cosmetic ingredients to the skin.

Users using such mask packs may have improved appearance and feel comfortable, and a mask pack composition having various ingredients in image regions may be provided. In addition, if base sheets having improved adhesion to cosmetic composition layers are provided, the base sheets may be widely used in related fields.

### [Disclosure]

### [Technical Problem]

An aspect of the present disclosure may provide a mask pack base sheet capable of making users feel better and having improved adhesion to a cosmetic composition layer.

Another aspect of the present disclosure may provide a mask pack using the mask pack base sheet.

### [Technical Solution]

According to an aspect of the present disclosure, a mask pack base sheet includes: a base sheet, and a single-layer image print part formed on at least one side of the base sheet.

According to another aspect of the present disclosure, a mask pack includes:
the mask pack base sheet, and a cosmetic composition layer.

According to the present disclosure, the mask pack base sheet may include an image having various shapes to make users feel better and may have improved adhesion to the cosmetic composition layer. In addition, a mask pack composition having various ingredients may be provided according to image regions and may be seen by users.

### [Description of Drawings]

FIG. 1 illustrates an example of a base sheet for a mask pack according to the present disclosure, the mask pack base sheet being a mesh sheet formed of a polymer resin.
FIG. 2 illustrates an example of a hydrogel mask pack, including the mask pack base sheet according to the present disclosure.

### [Best Mode]

Exemplary embodiments of the present disclosure will now be described in detail with reference to the accompanying drawings. The disclosure may, however, be exemplified in many different forms and should not be construed as being limited to the specific embodiments set forth herein.

The present disclosure provides a base sheet for a mask pack. More particularly, the present disclosure provides a mask pack base sheet including a base sheet and a single-layer image print part formed on at least one side of the base sheet.

In the present disclosure, the term "base sheet" may refer to a support to which a cosmetic composition layer, including effective ingredients to be transferred to the skin, may be applied, or a base which may be impregnated with such a cosmetic composition.

Particularly, the mask pack base sheet of the present disclosure includes the image print part formed on at least one side of the base sheet. The image print part may have a shape of at least one selected from the group consisting of animals, plants, characters, natural shapes, geometric patterns, and human faces. However, the shape of the image print part is not limited thereto. That is, the image print part may have any shape expressible in an image.

Furthermore, in the present disclosure, the image print part is formed in a single layer, that is, in one layer. For example, although some regions of the image print part are first formed and the other regions of the image print part are then formed, if the image print part is finally a one-layer part, the image print part is considered as being formed in a single layer, that is, not considered as being formed in two or more layers.

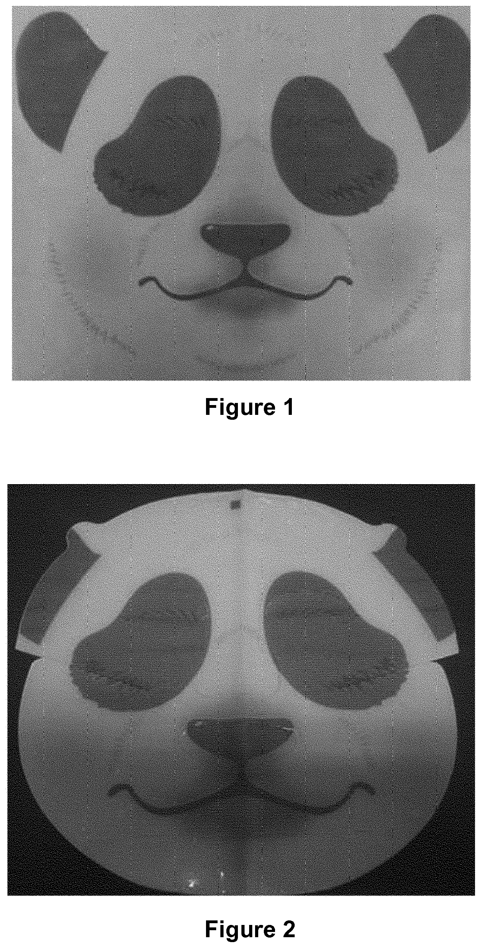

**FIGS. 1** and **2** illustrate an example of a mask pack base sheet, including an image print part implemented to have an animal shape, according to the present disclosure, FIG. 1 illustrating a mask pack base sheet having a panda's face shape, and FIG. 2 illustrating a hydrogel mask pack manufactured using the mask pack base sheet having a panda's face shape.

The mask pack base sheet of the present disclosure may be formed of at least one material selected from the group consisting of a polymer resin, a paper, a pulp, a nonwoven fabric, and a textile. For example, the mask pack base sheet may be a mesh sheet formed of the polymer resin.

The polymer resin may be selected from the group consisting of polyethylene terephthalate (PET), polyethylene (PE), polyester (PS), nylon, polypropylene (PP), oriented polypropylene (OPP), casting polypropylene (CPP), high density polyethylene (HDPE), low density polyethylene (LDPE), linear low density polyethylene (LLDPE), polyvinyl chloride, polyacrylonitrile, and polyurethane.

In addition, the textile may be formed of a natural fiber or a synthetic fiber. Examples of the natural fiber include rayon, silk, cotton, linen, etc., and examples of the synthetic fiber include nylon, polypropylene, polyethylene terephthalate, polybutylene terephthalate, polybutadiene, polyamide, polyethylene, polyvinyl alcohol, polyvinyl chloride, polyacrylonitrile, polyurethane, polylactic acid, etc.

According to the present disclosure, the base sheet may preferably be formed of a polymer resin film having a thickness of 0.1 mm to 5 mm and a surface roughness of 0.01 µm to 100 µm, and the polymer resin film may be implemented in the form of a mesh sheet. As described above, if the base sheet is a mesh sheet formed of a polymer resin film, a hydrogel layer or a cataplasm layer may be easily attached to at least one side of the base sheet as a cosmetic composition layer.

If the thickness of the base sheet is less than 0.1 mm, the base sheet may easily be damaged when the base sheet is stored, transferred, or used. If the thickness of the base sheet is greater than 5 mm, the mask pack may be excessively thick, and thus it may be difficult to bring the mask pack into contact with the face or attach the mask pack to the face.

The polymer resin film having a degree of surface roughness within the above-mentioned range may have a high degree of adhesion to a hydrogel layer and thus may more firmly fix the hydrogel layer, thereby preventing slippage between the hydrogel layer and the base sheet. If the surface roughness of the polymer resin film is less than 0.01 µm, the base sheet may have insufficient adhesion to a hydrogel layer. If the surface roughness of the polymer resin film is greater than 100 µm, the sharpness of a print image may be low, and the base sheet may irritate the skin.

Regions of the base sheet having a degree of surface roughness within the above-mentioned range may be uniformly or irregularly distributed. For example, regions of the base sheet having a degree of surface roughness within the above-mentioned range may be uniformly distributed for uniform adhesion to a cosmetic composition layer such as a hydrogel layer.

The image print part may be formed on the base sheet by any general printing method such as a silk screen printing method, a gravure printing method, a digital printing method, or an ultraviolet (UV) printing method.

Meanwhile, ink for this use may have a sublimation temperature within the range of 50°C to 600°C, preferably within the range of 140°C to 300°C, 150°C to 250°C, or 140°C to 250°C, and more preferably within the range of 190°C to 230°C, and printing may be performed within any temperature range included in any one of the above-mentioned temperature ranges. If the sublimation temperature of the ink is lower than 50°C, the base sheet may not be sufficiently dyed with the ink, and thus printing sharpness may be low, and if the sublimation temperature of the ink is higher than 600°C, the base sheet may melt at the sublimation temperature of the ink.

If the base sheet is formed of a polymer resin and an image is formed on the base sheet using a sublimation ink, the base sheet may be highly durable even though a multilayer print part or an additional coating layer is not formed.

In detail, a method of manufacturing a mask pack base sheet includes: a process of printing an image on transfer paper using a transfer ink; and a process of transferring the image from the transfer paper to a base sheet.

The printing process may be performed at room temperature by a gravure printing method or a digital printing method, and the room temperature may range from 20°C to 30°C.

According to the method of manufacturing a mask pack base sheet on which an image is formed according to the present disclosure, the transferring process may be performed by attaching the transfer paper and the base sheet to each other and pressing the attached transfer paper and the base sheet at a high temperature, for example, through a thermal press process or a thermal rolling press process.

At this time, the transfer paper and the base sheet are attached to each other by attaching the base sheet to a side of the transfer paper on which the image is printed, to transfer the image to the base sheet. For example, the transfer paper and the base sheet may be attached to each other for double printing on both sides. In detail, the transfer paper and the base sheet are arranged to come into contact with each other and are then pressed, for example, using thermal rollers at about 200°C for about 30 seconds, so as to transfer the transfer ink from the transfer paper to the base sheet.

Preferably, the transferring process may be performed within a temperature range of 140°C to 300°C with a pressing force of 50 kg to 200 kg. More preferably, the transferring process may be performed within a temperature range of 190°C to 230°C.

If the transferring process is performed at a temperature lower than 140°C, the image may not be smoothly transferred, and if the transferring process is performed at a temperature higher than 300°C, a support structure may burn or thermally deformed. In addition, if the pressing force in the transferring process is less than 50 kg, the image may not be smoothly transferred, and if the pressing force in the transferring process is greater than 200 kg, the support structure may be deformed.

For example, it may be preferable that the transferring process be performed at a temperature of about 200°C with a pressing force of about 100 kg for a period of 15 seconds to 30 seconds by using a high-temperature roller press.

The transfer ink may have an ink composition including a solvent, a disperse dye, a wetting agent, and a dispersing agent. In detail, the ink composition may include a solvent in an amount of 30 wt % to 70 wt %, a disperse dye in an amount of 0.5% to 15%, a wetting agent in an amount of 10 wt % to 50 wt %, and a dispersing agent in an amount of 0.1 wt % to 5 wt %.

In the present disclosure, the transfer ink includes a solvent, a disperse dye, a wetting agent, and a dispersing agent such that the transfer ink may be applied to the transfer paper (intermediate medium) and then transferred to the base sheet (permanent medium) by heat and pressure.

In the present disclosure, the disperse dye may undergo sublimation under the influence of heat, thereby dyeing the base sheet through a reaction caused by heat and pressure. In this case, the transfer ink may not be eluted to other parts.

In the present disclosure, the transfer ink, particularly the disperse dye, undergoes a thermal reaction and sublimation, thereby being transferred. The disperse dye may be, for example, amine and may include a weak hydrophilic group, such as a hydroxyl group, ether, or ester in its molecules. However, the disperse dye may not include a strong hydrophilic group such as a sulfone group. In more detail, the disperse dye may include azo, azomethyl, nitro, anthraquinone, quinoline, a coumarin dye, etc. The disperse dye may include only one of the listed substances or a combination of at least two of the listed substances.

Preferably, the content of the disperse dye may be within the range of 0.5 wt % to 15 wt % based on the total weight of the ink composition. If the content of the disperse dye is less than 0.5 wt %, colors may not be clearly present after printing, and if the content of the disperse dye is greater than 15 wt %, the disperse dye may not be easily dispersed and transferred. More preferably, the content of the disperse dye may be within the range of 1 wt % to 10 wt %.

The content of the solvent may be within the range of 30 wt % to 70 wt % based on the total weight of the ink composition. For example, the solvent may be water, such as purified water or deionized water, and may include a water-miscible organic solvent.

The organic solvent may include at least one of an alcohol-based solvent, a ketone-based solvent, a polyhydric alcohol-based solvent, a nitrogen-containing solvent, dimethyl sulfoxide, tetramethyl sulfone, and a sulfur-containing compound of thioglycol.

In this case, an aqueous polymer may be additionally included. The aqueous polymer may include a polymer having a urethane group, a copolymer having a urethane group, a polymer having an acryl group, a copolymer having an acryl group, and a mixture thereof. For example, the aqueous polymer may include a urethane-containing copolymer, an acryl polymer, a urethane-acrylic copolymer blended with an acryl polymer, a blend of at least one polyurethane polymer and at least one acryl polymer, a blend of at least two acryl polymers, and a blend thereof.

When the disperse dye disperses in the form of nano-sized particles, the nano-sized particles tend to clump together to reduce surface energy thereof. At this time, the aqueous polymer surrounds the nano-sized particles and reduces the surface energy of the nano-sized particles. Therefore, the nano-sized particles of the disperse dye are hindered from clumping together, and the dispersion of the disperse dye is facilitated.

The content of the dispersing agent is within the range of 0.1 wt % to 5 wt %, and a material having a high degree of high-temperature dispersibility may be used as the dispersing agent. For example, a lignin sulfonic acid salt, a condensation product of lignin sulfonic acid and formaldehyde, a condensation product of alkyl naphthalene sulfonic acid and formaldehyde, or a condensation product of creosote oil sulfonic acid and formaldehyde may be used as the dispersing agent, and the dispersing agent may be an anionic dispersing agent.

The anionic dispersing agent is not limited to a particular material. For example, the anionic dispersing agent may be a high-molecular condensation product of a sulfonic acid such as an aromatic sulfonic acid and formalin, or a high-molecular condensation product of lignin sulfonic acid and formalin. Examples of the condensation product of an aromatic sulfonic acid and formalin include: a condensation product of creosote oil sulfonic acid and formalin; a condensation product of cresol sulfonic acid and formalin; a condensation product of phenol sulfonic acid and formalin; a condensation product of β-naphthol sulfonic acid and formalin; a condensation product of β-naphthalene sulfonic acid, β-naphthol sulfonic acid, and formalin; and a condensation product of cresol sulfonic acid, 2-naphthol-6-sulfonic acid, and formalin. All of the listed condensation products include a salt such as a sodium salt. Particularly, the anionic dispersing agent may be a condensation product of creosote oil sulfonic acid and formalin, a condensation product of naphthalene sulfonic acid and formalin, or a condensation product of lignin sulfonic acid and formalin.

A product available on the market may be used as the dispersing agent, and examples of the condensation product of an aromatic sulfonic acid (salt) and formalin available on the market include DEMOL N (a product by Kao Corp.), DEMOL C (a product by Kao Corp.), and DEMOL SNB (a product by Kao Corp.) which is a sodium salt of a special condensation product of an aromatic sulfonic acid and formalin.

In the present disclosure, the content of the wetting agent of the transfer ink is within the range of 10 wt % to 50 wt %. If the content of the wetting agent is less than 10 wt %, the ink composition dries at a fast rate, and thus the ink composition may dry in a nozzle of an inkjet head and may clog the nozzle. If the content of the wetting agent is greater than 50 wt %, the viscosity of the ink composition may be high, and the dispersibility of the ink composition may be negatively affected. Preferably, the content of the wetting agent may be within the range of 20 wt % to 40 wt %.

Examples of the wetting agent include glycerol, sugars, glycols, etc. In more detail, the wetting agent may be a polyhydric alcohol such as ethylene glycol, diethylene glycol, triethylene glycol, propylene glycol, tetraethylene glycol, polyethylene glycol, glycerol, 2-methyl-2,4-pentanediol, 1,2,6-hexanetriol, or thioglycol.

In addition, examples of the wetting agent include lower alkyl mono-ethers or diethers derived from alkylene glycols, such as ethylene glycol mono-methyl or mono-ethyl ether, diethylene glycol mono-methyl or mono-ethyl ether, propylene glycol mono-methyl or mono-ethyl ether, triethylene glycol mono-methyl or mono-ethyl ether, diethylene glycol dimethyl or di-ethyl ether, diethylene glycol monobutylether, etc.

In addition, if required, the ink composition of the present disclosure may include various additives. Examples of the additives include surfactants, germicides, viscosity controlling agents, pH adjusting agents, buffering agents, antioxidants, conductivity changing agents, etc. However, the additives are not limited thereto.

The image may be smoothly printed on the transfer paper using the above-described transfer ink composition, and then the image may be transferred from the transfer paper to the base sheet in the next transferring process.

In particular, the above-described transfer ink is suitable for a transferring process performed by a high-temperature pressing method, and if the base sheet includes a polymer compound resin, the transfer ink may be more effectively transferred and fixed, without problems such as elution.

The transfer paper may be a thermal transfer paper or may be any kind of transfer paper used in the related art.

According to the present disclosure, the transfer paper and the transfer ink are used, the printing process is performed by a gravure method and a digital output method, and then the transferring process is performed. Therefore, the shape of the image is not limited to a particular width, and printing may be performed on the entire area of the transfer paper according to a preset width of a copper plate in a gravure printing process.

According to the present disclosure, if required, after the method of manufacturing a mask pack base sheet having an image formed thereon is performed, a press punching process or a continuous coating process including a Thompson punching process may be formed for mass production. In the case of an essence mask pack, the Thompson punching process may be performed after a content impregnation process, and in the case of a hydrogel mask pack, the Thompson punching process may be performed after a content coating process. In addition, if an eyemark is printed on an edge region together with a particular image, each mask pack obtained by a subsequent cutting process may be more precisely punched in the same pattern of holes.

According to the present disclosure, a mask pack base sheet on which an image is formed may be obtained, and such mask pack base sheets may be efficiently, continuously produced by the above-described manufacturing method. If an image is formed by a high-temperature pressing transfer method, ink may be securely fixed to a base sheet and may not be absorbed in other layers. Particularly, according to the method of manufacturing a base sheet of the present disclosure, a desired image may be effectively formed even on a nonwoven fabric or mesh type base sheet.

Another aspect of the present disclosure provides a mask pack including the above-described mask pack base sheet and a cosmetic composition layer.

According to the present disclosure, for example, the mask pack is an image mask pack including a mask pack base sheet and a cosmetic composition layer, wherein a single-layer image print part is formed on at least one side of the base sheet, and the base sheet is formed of a polymer resin film selected from the group consisting of polyethylene terephthalate (PET), polyethylene (PE), polyester (PS), nylon, polypropylene (PP), oriented polypropylene (OPP), casting polypropylene (CPP), high density polyethylene (HDPE), low density polyethylene (LDPE), and linear low density polyethylene (LLDPE). The image print part is formed of a sublimation ink having a sublimation temperature within the range of 140°C to 250°C.

The cosmetic composition layer may be a hydrogel layer or a cataplasm layer attached to at least one side of the base sheet, or the cosmetic composition layer may be included in the mask pack of the present disclosure by impregnating at least a portion of the base sheet with a cosmetic composition. In the latter case, in which the cosmetic composition layer is provided by impregnating at least a portion of the base sheet, although the cosmetic composition layer may not be physically distinguished from the base sheet, the cosmetic composition layer is distinctly referred to in the present disclosure for ease of description.

The cataplasm layer may include a hydrophilic polymer, a skin-effective ingredient, a polyhydric alcohol, and a crosslinking agent. In addition, if required, the cataplasm layer may further include another functional additive.

The hydrophilic polymer may include at least one selected from the group consisting of gelatin, polyacrylate or a salt thereof, polymethacrylic acid, carboxyvinyl polymer, polyvinyl alcohol, polyacrylamide, polyethylene oxide, polyethyleneimine, polyvinylpyrrolidone, methyl cellulose, cellulose, carboxymethyl cellulose, and hydroxyethyl cellulose.

The polyhydric alcohol may include at least one selected from the group consisting of glycerin, ethylene glycol, propylene glycol, dipropylene glycol, and butylene glycol.

The cross-linking agent may be a polyvalent metal salt. For example, the cross-linking agent may include at least one selected from the group consisting of aluminum hydroxide, aluminum hydroxide gel, water-containing aluminum silicate, kaolin, aluminum acetate, aluminum lactate, aluminum stearate, calcium chloride, magnesium chloride, aluminum chloride, magnesium aluminosilicate, and magnesium aluminometasilicate.

The skin-effective ingredient may include: an ingredient having a positive effect on the skin such as anti-wrinkling, whitening, ultraviolet blocking, anti-aging, moisturizing, or tightening; and a medication for preventing and curing skin diseases. The skin-effective ingredient is not particularly limited as long as the skin-effective ingredient has a positive effect on the skin.

For example, the skin-effective ingredient may include at least one selected from the group consisting of retinol, retinyl palmitate, retinyl acetate, retinoic acid, coenzyme Q10, elastin, collagen, hyaluronic acid, ceramide, collagen, caffeine, chitosan, ascorbic acid, ascorbyl glucoside, alpha-bisabolol, tocopherol, tocopherol acetate, arbutin, niacinamide, adenosine, retinol acetate, vitamins A, D, and E, and a natural extract.

The natural extract may include at least one extracted from the group consisting of aloe, green tea, ginseng, red ginseng, pearl, wood vinegar, pine needles, ginkgo leaves, propolis, mulberry leaves, silkworms, snail slime, kakadu plum, camu-camu, acai palm, squalane, caviar, broccoli, blueberries, witch hazel, acerola, chlorella, mangosteen, guava, cornus officinalis, carrots, caffeine, hamamelis, spirulina, salmon roe, ecklonia cava, giant kelp, laminariae thallus, portulaca oleracea, green laver, agar, mulberry tree roots, raspberry, wild strawberry, sea weed fusiforme, sargassum, edelweiss, chamomile, lavender, peppermint, eucalyptus, lemon balm, oregano, tea tree, scutellariae radix, houttuynia cordata, sea buckthorn, and citron citrus.

In addition, the functional additive may include at least one selected from the group consisting of preservatives, thickeners, perfumes, humectants, surfactants, and oils.

The hydrogel layer may include a gelation polymer, a polyhydric alcohol, water, and a skin-effective ingredient. In addition, if required, the hydrogel layer may further include another functional additive. However, the hydrogel layer is not limited thereto. The hydrogel layer may include water in an amount of 20% to 90%, exhibiting high water content characteristics. The hydrogel layer may include various hydrogels used for mask packs.

For example, preferably, the gelation polymer may include at least one branched gelation polymer (water-soluble polysaccharide polymer) selected from the group consisting of galactomannan, glucomannan, guar gum, locust bean gum, and Pluronic in an amount of 0.01 wt % to 10 wt %; and at least one electrolytic gelation polymer (polysaccharide electrolyte polymer) selected from the group consisting of agar, algin, carrageenan, xanthan, and gellan, in an amount of 0.01 wt % to 10 wt %.

The polyhydric alcohol and the skin-effective ingredient are the same as those explained in the description of the cataplasm layer.

In the mask pack in which the hydrogel layer is formed on at least one side of the base sheet, the base sheet may be a mesh sheet formed of a polymer resin. Particularly, when the viscosity of the hydrogel layer is relatively low, the mask pack may be useful.

Furthermore, in the mask pack of the present disclosure in which the base sheet is attached to a side of the hydrogel layer, a release film may be additionally attached to the other side of the hydrogel layer. In this case, the release film may function as a protection film, and the hydrogel layer may be stably maintained.

The mask pack base sheet may include an image print part formed of at least one closed figure. In this case, particularly, the hydrogel layer may include at least one skin-effective ingredient included inside the closed figure, and different from a skin-effective ingredient included outside the closed figure.

For example, referring to FIG. 1, in the image print part having a panda's face shape, the panda's ears have a closed figure shape, and, referring to FIG. 2, the panda's eyes of the panda's face shape have a closed figure shape. The panda's ears illustrated in FIG. 1 may be portions on which hydrogel regions having a hydrogel composition particularly useful for skin regions to be brought into contact with the panda' ears are formed, and the panda's eyes illustrated in FIG. 2 may be portions on which hydrogel regions having a hydrogel composition particularly useful for skin regions to be brought into contact with the panda's eyes are formed.

In this manner, regions having various ingredients may be formed on the image print part, based on boundaries formed by the image print part. Therefore, although the mask pack is used once, different skin regions may be respectively provided with various ingredients having multiple effects at the same time, and a user may visually check the effects of various ingredients of the mask pack composition according to image regions. Therefore, ingredients may be properly used according to regions of the face.

According to the present disclosure, for example, a mask pack may be manufactured through a process of printing an image on a base sheet, a process of winding the base sheet around a roll after drying and deodorizing the base sheet, and a process of forming a cosmetic composition on one or both sides of the base sheet by a coating or application method while rewinding the base sheet. As explained in the description of the method of manufacturing a mask pack base sheet, for example, the printing process may include a process of printing an image on transfer paper using a transfer ink; and transferring the image from the transfer paper to the base sheet.

In addition, if required, the mask pack base sheet of the present disclosure may include openings in eye, nose, and mouth portions. In this case, a process of forming openings by punching the mask pack base sheet according to the printed image may be additionally performed.

Furthermore, according to the present disclosure, a process of sealing the mask pack using a pouch may be additionally performed for the sale and distribution of the mask pack.

Furthermore, during the mask pack manufacturing processes, if an eyemark is printed on an edge region together with a particular image in the printing process, each mask pack obtained by a subsequent cutting process may be more precisely punched in the same pattern of holes.

Hereinafter, the present disclosure will be described more specifically through examples. The following examples are for illustrative purposes only and are not intended to limit the scope of the present invention.

### [Mode for Invention]

### 1. Preparation of an ink composition

An ink composition was prepared by mixing anthraquinone dye in an amount of 10 wt %, glycerol as a wetting agent in an amount of 10 wt %, lignosulfonate sodium salt as a dispersing agent in an amount of 5 wt %, and the balance of an ethanol solvent.

### 2. Fabrication of a mask pack having an image

### Example 1

An image was printed on transfer paper by a digital method using the ink prepared in Section 1. A mesh type PET support having a thickness of 0.2 mm was put on a side of the transfer paper on which the image was formed, and the PET support and the transfer paper were passed through a thermal press using a roll press having a temperature of 230°C while applying a pressing force of 100 kg, so as to transfer the image to the PET support.

Next, both sides of the PET support were coated with a hydrogel composition containing a gelation polymer to a thickness of 950 g/sqm, and the PET support was cut in a shape corresponding to the image by using a Thompson punching machine. In this manner, a mask pack was fabricated.

### Example 2

A mask pack was fabricated in the same manner as in Example 1, except that both sides of a PET nonwoven fabric support on which an image was printed were coated with cataplasm layers to a thickness of 1,000 g/sqm.

### Example 3

A mask pack was fabricated in the same manner as in Example 1 except that both sides of a polypropylene nonwoven fabric support on which an image was printed were coated with cataplasm layers to a thickness of 1,000 g/sqm.

### Example 4

An image was printed on a side of a PET support having a thickness of 0.2 mm and a surface roughness of 80 µm by using an ink having a sublimation temperature of 140°C and a UV radiation method. Both sides of the PET support on which the image was printed were coated with a hydrogel composition including carrageenan in an amount of 5 wt % and locust bean gum (LBG) in an amount of 5 wt %, and the PET support was cut in a shape corresponding to the image using a Thompson punching machine. In this manner, a hydrogel mask pack was fabricated.

### Example 5

An image was printed on a side of a PET support having a thickness of 0.5 mm and a surface roughness of 100 µm by using an ink having a sublimation temperature of 200°C and a gravure method. Both sides of the PET support on which the image was printed were coated with a hydrogel composition including carrageenan in an amount of 5 wt % and locust bean gum (LBG) in an amount of 5 wt %, and the PET support was cut in a shape corresponding to the image using a Thompson punching machine. In this manner, a hydrogel mask pack was fabricated.

### Comparative Example 1

A mask pack was fabricated in the same manner as in Example 1 except that a cotton nonwoven fabric support was used instead of the PET support.

### Comparative Example 2

A mask pack was fabricated in the same manner as in Example 2 except that an ink having a transfer temperature of 50°C was used.

### Comparative Example 3

A mask pack was fabricated in the same manner as in Example 4 except that the surface roughness of the PET support was 1,000 µm.

### Comparative Example 4

A mask pack was fabricated in the same manner as in Example 4 except that an ink having a sublimation temperature of 30°C was used.

### 3. Evaluation of printing sharpness and spreading/peeling

The printing sharpness and quality of the mask packs fabricated in Examples 1, 2, and 3, and Comparative Examples 1 and 2 were evaluated by comparing the mask packs with the naked eye, and results thereof are shown in Table 1 below.

**[Table 1]**

| | Example 1 | Example 2 | Example 3 | Comparative Example 1 | Comparative Example 2 |
|---|---|---|---|---|---|
| Printing Sharpness | ⊚ | ⊚ | ⊚ | X | X |
| Spreading/peeling | ⊚ | ⊚ | ⊚ | X | X |

| | | | | | |
|---|---|---|---|---|---|
| *Evaluation -⊚: normal, ○: satisfactory, Δ: poor, X: very poor | | | | | |

### 4. Evaluation of dyeing uniformity and printing sharpness

The dyeing uniformity and printing sharpness of the images printed on the hydrogel mask packs were evaluated with the naked eye, and results thereof are shown in Table 2, below.

**[Table 2]**

| | Example 4 | Example 5 | Comparative Example 3 | Comparative Example 4 |
|---|---|---|---|---|
| Dyeing Uniformity | ⊚ | ⊚ | X | X |
| Printing Sharpness | ⊚ | ⊚ | Δ | X |

| | | | | |
|---|---|---|---|---|
| *Evaluation - very good: ⊚, good: ○, poor: Δ, very poor: X | | | | |

Dyeing uniformity was evaluated as being very good (⊚) if ink peeling and stains were not observed at all, being good (○) if ink peeling and stains were slightly observed, being poor (Δ) if ink peeling occurred in about 3% of a printed area, and being very poor (X) if ink peeling occurred in about 10% of a printed area. Particularly, it could be seen that ink peeling occurred in Comparative Example 4.

Printing sharpness was evaluated as being very good (⊚) if the printed image was the same as the original, being poor (Δ) if the printed image was blurred, compared to the original, and being very poor (X) if the printed image was blurred and difficult to perceive with the naked eye.

### 5. Dye elution test

Before a base sheet on which an image was printed was coated with a cosmetic composition, the base sheet was tested by color fastness to hot water, KS K 0216 (30 x 30 cm) (put in boiling water for 60 minutes), and dye discoloration was checked. The results thereof are shown in Table 3, below.

**[Table 3]**

| | Example 1 | Example 2 | Example 3 | Comparative Example 1 | Comparative Example 2 |
|---|---|---|---|---|---|
| Evaluation grade | 5 | 5 | 5 | 3 | 3 |
| | Normal | Normal | Normal | Dye blurred | Dye blurred |

The test results were obtained by observing a base sheet on which an image was printed before and after the test. After the test, Grade 5 indicates a good state, in which dye discoloration hardly occurred, and Grade 1 indicates a poor state, in which dye discoloration and decoloration occurred severely.

### 6. Skin irritation test

The mask packs fabricated in the examples and comparative examples were attached to the forearms of fifty male and female subjects and covered with plastic caps according to a closed patch test method, and after one hour, irritation to the skin was evaluated. The degree of irritation was evaluated as follows.
0: no irritation
1: slight irritation
2: erythema
3: irritation followed by severe erythema, edema, etc.
4: irritation followed by very severe erythema, edema, etc.

After the degree of irritation to the skin of each subject was measured, the sum of measured values was divided by the number of the subjects to calculate an average irritation degree, as shown in Table 4.

**[Table 4]**

| | Example 1 | Example 2 | Example 3 | Comparative Example 1 | Comparative Example 2 |
|---|---|---|---|---|---|
| Average | 0 | 0 | 0 | 2.5 | 3.0 |

The results shown in Table 4, above, are average values of irritation calculated by the above-mentioned evaluation method, and the results show that the mask packs fabricated according to the present disclosure cause almost no irritation to the skin.

While exemplary embodiments have been shown and described above, the scope of the present invention is not limited thereto, and it will be apparent to those skilled in the art that modifications and variations could be made without departing from the scope of the present invention, as defined by the appended claims.

## Claims

1. Mask pack base sheet comprising:
a base sheet; and
a single-layer image print part formed on at least one side of the base sheet.

2. Mask pack base sheet according to claim 1, wherein the image print part has a shape of at least one selected from the group consisting of animals, plants, characters, natural shapes, geometric patterns, and human faces.

3. Mask pack base sheet according to claim 1, wherein the base sheet comprises at least one selected from the group consisting of a polymer resin, a paper, a pulp, a nonwoven fabric, and a textile.

4. Mask pack base sheet according to claim 3, wherein the polymer resin is selected from the group consisting of polyethylene terephthalate (PET), polyethylene (PE), polyester (PS), aluminum, nylon, polypropylene (PP), oriented polypropylene (OPP), casting polypropylene (CPP), high density polyethylene (HDPE), low density polyethylene (LDPE), and linear low density polyethylene (LLDPE).

5. Mask pack base sheet according to claim 1, wherein the base sheet is formed of a polymer resin film having a thickness of 0.1 mm to 5 mm and a surface roughness of 0.01 µm to 100 µm.

6. Mask pack base sheet according to claim 1, wherein the single-layer image print part is formed using a sublimation ink having a sublimation temperature within a range of 140°C to 250°C.

7. Mask pack comprising:
the mask pack base sheet according to any one of claims 1 to 6; and
a cosmetic composition layer.

8. Mask pack according to claim 7, wherein the cosmetic composition layer is a hydrogel layer or a cataplasm layer formed on at least one side of the base sheet.

9. Mask pack according to claim 7, wherein at least a portion of the base sheet is impregnated with the cosmetic composition layer.

10. Mask pack according to claim 8, wherein the cataplasm layer comprises a hydrophilic polymer, a skin-effective ingredient, a polyhydric alcohol and a crosslinking agent.

11. Mask pack according to claim 10, wherein the hydrophilic polymer comprises at least one selected from the group consisting of gelatin, polyacrylate or a salt thereof, polymethacrylic acid, carboxyvinyl polymer, polyvinyl alcohol, polyacrylamide, polyethylene oxide, polyethyleneimine, polyvinylpyrrolidone, methyl cellulose, cellulose, carboxymethyl cellulose, and hydroxyethyl cellulose.

12. Mask pack according to claim 8, wherein the hydrogel layer comprises a gelation polymer, a polyhydric alcohol, water, and a skin-effective ingredient.

13. Mask pack according to claim 12, wherein the gelation polymer comprises:
at least one branched gelation polymer in an amount of 0.01 wt % to 10 wt %, the branched gelation polymer being a water-soluble polysaccharide polymer selected from the group consisting of galactomannan, glucomannan, guar gum, locust bean gum, and Pluronic®; and
at least one electrolytic gelation polymer, in an amount of 0.01 wt % to 10 wt %, the electrolytic gelation polymer being a polysaccharide electrolyte polymer selected from the group consisting of agar, algin, carrageenan, xanthan, and gellan.

14. The mask pack according to claim 7, wherein the mask pack base sheet comprises an image print part formed of at least one closed figure.

15. The mask pack according to claim 8, wherein the hydrogel layer comprises at least one skin-effective ingredient included inside the closed figure and different from a skin-effective ingredient included outside the closed figure.
